Europäisches Patentamt

(19) European Patent Office

Office européen des brevets

(11) Publication number: **0 084 960**
**B1**

(12) **EUROPEAN PATENT SPECIFICATION**

(45) Date of publication of patent specification: **05.11.86**

(51) Int. Cl.⁴: **A 61 F 13/20,** A 61 F 13/12

(21) Application number: **83300289.2**

(22) Date of filing: **20.01.83**

(54) Epistaxis sponge.

(30) Priority: **27.01.82 US 343234**

(43) Date of publication of application:
**03.08.83 Bulletin 83/31**

(45) Publication of the grant of the patent:
**05.11.86 Bulletin 86/45**

(84) Designated Contracting States:
**AT BE CH DE FR GB IT LI LU NL SE**

(56) References cited:
**DE-B-2 554 596**
**US-A-1 133 770**
**US-A-1 555 708**
**US-A-1 804 670**
**US-A-2 362 704**
**US-A-3 443 562**
**US-A-3 595 236**

(73) Proprietor: **Rangaswamy, Avvari**
**P.O. Box 426 13 Eastern Drive**
**Littleton North Carolina 27850 (US)**

(72) Inventor: **Rangaswamy, Avvari**
**P.O. Box 426 13 Eastern Drive**
**Littleton North Carolina 27850 (US)**

(74) Representative: **Dearing-Lambert, Peter Richard**
**Dearing-Lambert & Co. 18 Main Street Market**
**Bosworth**
**Nuneaton Warwickshire CV13 0JW (GB)**

Courier Press, Leamington Spa, England.

## Description

Background of the invention

Epistaxis or nose bleed while quite common and generally experienced by everyone at one time or another is at best an inconvenient and often disturbing experience. Even though considerable advances have been achieved in the treatment of a wide variety of disorders, the time honored methods for controlling anterior nose bleeds remain relatively unimproved. Simply tilting the head back, using cold compresses, squeezing the anterior part of the nose, i.e. the septum, and finally packing the nasal cavity are employed as standard epistaxis inhibiting techniques. The drawbacks to these techniques are personally known to almost everyone and in particular the discomfort of squeezing the nose with the resultant need for breathing through the mouth. In the case of packing, there is a need for special instruments and professional expertise when both packing and unpacking the nasal cavity.

Nose bleeds can occur for a wide variety of reasons including rheumatic fever, infectious mononucleosis, sickel cell anemia, systemic haemorrhagic disorders and hypertension (in adults), hemangiomas, hereditary telangiectasis and angio fibromas. The most common causes of epistaxis in children arise in the nasal cavity, for example, by excessive drying, which can be traumatized by such activities as nose picking. The most common location for such varicosities is on the nasal septum above the nasal floor and approximately 0.5 cm inside the nose. This is characterised as Kiesselbauch's area and is the anastomotic site for a number of arterioles. Traumatising a varicosity in this area sufficiently to produce epistaxis is commonly referred to as an anterior nose bleed.

A nasal insert is known from U.S. Specification No. 1133770. Cotton tufts attached to opposite ends of a wire yoke can be inserted in respective nostrils when the yoke is bent to U-shape. One of the purposes of the nasal tampon of this Specification is as an applicator for any desired remedy. Another is to act as an air filter. The device would not be suitable for the effective treatment of epistaxis because the "pledgets" are not specially shaped to fit the nostrils prior to insertion and when inserted will contact the nasal cavities only in the regions of the outer portions of the tufts of lint or cotton. Because they will make substantially only a line contact with the interiors of the nostrils, where the outer portions of the tufts undergo deformation, the pledgets will irritate a traumatised nasal cavity when inserted and removed and will not be effective to promote blood clotting over substantial areas of the cavity interiors.

Catemenial devices are known from U.S. Specifications Nos. 3595236 and 1555708. There is no suggestion that either of these should be used for the treatment of epistaxis and apart from other considerations each would be unsuitable in that it lacks a semi-rigid handle whereby it could be manipulated during insertion into and removal from a nasal cavity.

Specification No. PCT/SE80/00130 discloses a device for temporarily or permanently closing body passages such as blood vessels, ureters, testicular ducts and oviducts. A member which is stiff and/or hard in the unswollen state is introduced into a considerably larger body passage and then swells to block the passage, becoming soft as its does so. It is stated that the original geometrical shape of the member is not critical as when swollen and softened by the absorbtion of body fluids it will conform to the shape of the passage in which it is inserted. The device of this Specification would not be suitable for the treatment of epistaxis because the insertion of an originally stiff or hard member would cause irritation and the time required for the member to swell would not afford the necessary immediate relief. More importantly, however, it is proposed that to prevent dislodgement of the member before it has swollen it should be provided with resilient projections to engage the wall of the body passage. Such projections would be wholly unacceptable in a device for the treatment of epistaxis for obvious reasons.

The invention as claimed is intended to overcome the deficiencies of previous proposals for the treatment of epistaxis by the provision of a device which comprises a pad adapted to promote blood clotting by having a shape initially conforming generally to that of the nasal cavity into which it is to be inserted so that the pressure of a fluid absorbent material is gently applied to a substantial traumatised area immediately upon insertion. The provision of a semi-rigid handle gives the device maximum adaptability and facilitates its gentle but effective manipulation.

An embodiment of the invention will now be described by way of example with reference to the accompanying drawings, in which:

Fig. 1 is a perspective view of the device of the present invention;

Fig. 2 is an end view in the direction of a longitudinal axis or of a handle shown in Fig. 1; and

Fig. 3 is a partial cross sectional view taken along the lines and arrows 3—3 in Fig. 1.

Detailed description of the drawings

The device of the present invention is characteristically constructed around a semi-rigid, flexible stem 1 which is provided with a gripping portion or handle 2 and an anchor 3. The length of the stem can vary as well as the specific shape of the handle and the configuration of the anchor depending on the functional requirements and physical properties of the materials selected. Of importance, is the requirement that the stem 1 be both rigid and yet flexible enough for manipulation during insertion of the shaped absorbent tip into the nasal cavity and removal therefrom while providing some support for the remainder of the structure. In the embodiment shown, a thin wire stem is provided which provides the necessary functionality when absorbent cotton 4 is utilized as the core material of a pad surrounded with at least one and preferably more than one cotton gauze outer wrapping 5 of the pad.

It is of course possible to substitute suitable plastics materials for the metal stem. Likewise, different handle and anchoring configurations can be employed without the need for extensive experimentation. The cottom gauze and absorbent cotton may also be replaced with suitable natural or synthetically manufactured absorbent materials as long as the finished device retains the capacity to generally hold its shape and preferably expand slightly when absorbing blood through the outer surface and into the interior of the device.

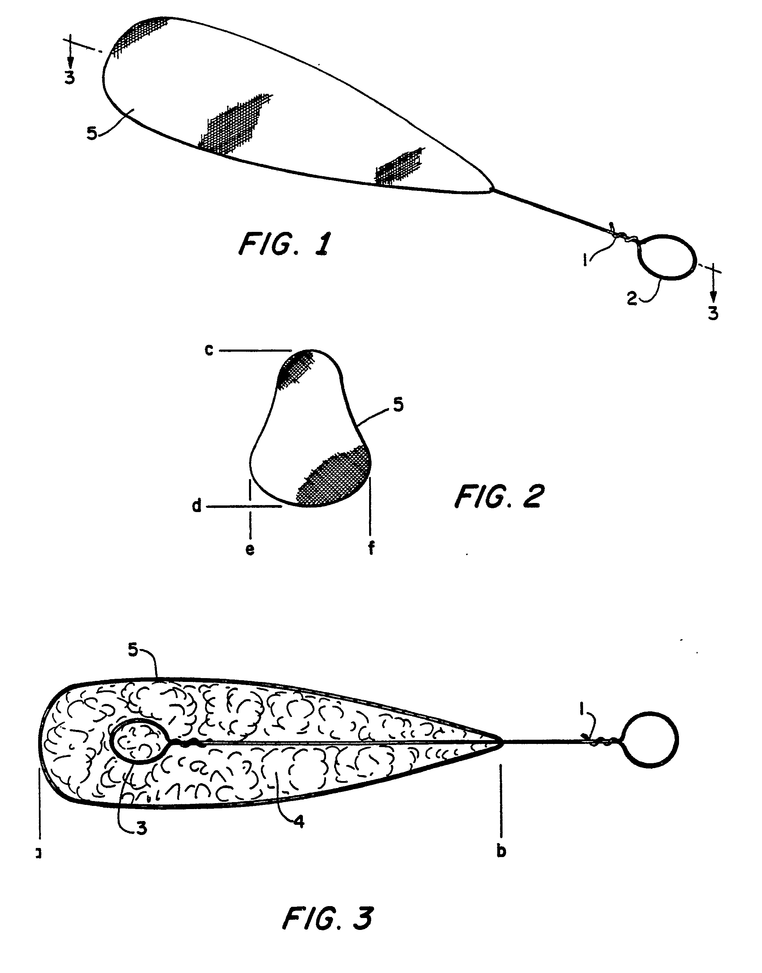

In the embodiment shown, conventional absorbent cottom 4 is provided as the core supported by the metal stem 1 and its associated anchor 3 and surrounded typically by several layers of cotton gauze 5 to form generally the shape shown in Figs. 1 and 2.

For optimum results, the dimension a—b (Fig. 3) should be approximately 4 cm to be suitable for an adult. Several sizes can, of course, be constructed, i.e. 3.5 cm, 3 cm and 2c, to accommodate differences in patient anatomy. The most preferred dimensions will approximately bear the following relative relationships in the embodiment described:

| Length | Height | Width |
|---|---|---|
| a—b 4 cm | c—d 2.5 cm | e—f 1.25 cm |
| 3—5 cm | 2.25 cm | 1.125 cm |
| 3.0 cm | 2.0 cm | 1.0 cm |
| 2.0 cm | 1.5 cm | 0.75 cm |

The anteroposterior dimension (c—d) and the transverse dimension (e—f) are important for both adaptation and a comfortable fit for the device into the nasal cavity. It is a particularly important aspect of the present invention that the pear-shaped cross section be maintained as close as is practical to the pear-like shape of the vestibule of the anterior nasal cavity. In general, a width (e—f), measured laterally or transversely of the width at a point below the mid-point of the cross-section shown in Fig. 2, would be approximately one-half the height (c—d) of the article.

The epistaxis sponge of the present invention can also be used to sponge or mop or provide control of bleeding points in the mucosa of the nasal cavity during minor surgery.

This invention has been described with reference to its current preferred embodiment, however, it will be appreciated that a wide variation in material selection is possible and other materials may be substituted and obtain the functional advantages disclosed herein. Likewise, further modifications are possible, as for example, the incorporation of soluble medicaments in the absorbent material or on the surface thereof to aid on coagulation, the prevention of infection or the like, without detracting from the function of the device, where such additives are a contemplated improvement for either a general or specialized function.

**Claims**

1. A device for insertion into a hemorrahging nasal cavity to treat epistaxis comprising a fluid absorbent pad (4, 5) and a semi-rigid handle (1, 2, 3) extending from one end of the pad (4, 5) for inserting the pad (4, 5) into and removing it from the nasal cavity characterised in that in a plane to which the longitudinal axis of the device is perpendicular the pad (4, 5), prior to its insertion in the nasal cavity, has a generally pear-shaped cross section, said section having a maximum width (e—f) below the mid point of its height (c—d) and said height (c—d) being approximately twice said maximum width (e—f) so that the pad (4, 5) is adapted for the absorption of fluid and to apply pressure to the traumatised mucosa when inserted in the cavity.

2. device as claimed in claim 1, characterised in that the handle (1, 2, 3) is anchored in the fluid absorbent pad (4, 5) and extends lengthwise beyond the pad (4, 5), a sufficient distance to be capable of being grasped externally of the nasal cavity when the pad (4, 5) is received therein, thereby providing for ease of manipulation of the pad (4, 5) and its insertion into and removal from the cavity.

3. A device as claimed in either of the preceding claims, characterised in that the pad (4, 5) is adapted resiliently to expand overall when absorbing fluid thereby increasing the pressure applied by the pad to the nasal mucosa as fluid is absorbed.

4. A device as claimed in any one of the preceding claims, characterised in that the pad (4, 5) comprises a core (4) of absorbent cotton surrounded by at least one layer (5) of cotton gauze.

**Patentansprüche**

1. Vorrichtung zum Einsetzen in eine blutende Nasenhöle, um Nasenbluten zu behandeln, umfassend ein Fluid absorbierendes Kissen (4, 5) und einen halbstarren Handgriff (1, 2, 3), der sich von einem Ende des Kissens (4, 5) erstreckt, um das Kissen (4, 5) in die Nasenhöhle einzusetzen und aus dieser zu entfernen, dadurch gekennzeichnet, daß in einer Ebene, zu welcher die Längsachse der Vorrichtung rechtwinklig verläuft, das Kissen (4, 5) vor seinem Einsetzen in die Nasenhöhle einen allgemein birnenförmigen Querschnitt hat, der seine maximale Breite (e—f) unter dem Mittelpunkt seiner Höhe (c—d) hat, und die Höhe (c—d) angenähert das Zweifache der maximalen Breite (e—f) beträgt, so daß das Kissen (4, 5) geeignet ist dafür, Fluid zu absorbieren und Druck an die verletzte Schleimhaut anzulegen, wenn es in den Hohlraum eingesetzt wird.

2. Vorrichtung nach Anspruch 1, dadurch gekennzeichnet, daß der Handgriff (1, 2, 3) in dem Fluid absorbierenden Kissen (4, 5) verankert ist und sich in Längsrichtung über das Kissen (4, 5) hinaus über eine ausreichende Strecke erstreckt, so daß er außerhalb des Nasenhohlraumes erfaßt werden kann, wenn das Kissen (4, 5) darin aufgenommen ist, so daß bequeme Handhabung des Kissens (4, 5) und bequemes Einsetzen in den Hohlraum und bequemes Entfernen aus dem Hohlraum geschaffen sind.

3. Vorrichtung nach einem der vorherhegenden Ansprüche, dadurch gekennzeichnet, daß das Kissen (4, 5) so ausgeführt ist, daß es sich insgesamt ausdehnen kann, wenn es Fluid absorbiert, so daß der durch das Kissen an die Nasenschleimhaut angelegte Druck erhöht wird, wenn Fluid absorbiert wird.

4. Vorrichtung nach irgendeinem der vorhergehenden Ansprüche, dadurch gekennzeichnet, daß das Kissen (4, 5) einen Kern (4) aus absorbierender Baumwolle aufweist, der von wenigstens einer Lage (5) aus Baumwollgaze umgeben ist.

**Revendications**

1. Un dispositif destiné à être introduit dans une cavité nasale présentant une hémorragie pour traiter l'épistaxis comprenant un tampon (4, 5) absorbant les liquides et une poignée (1, 2, 3) semi-rigide dépassant d'un extrémité du tampoin (4, 5) pour l'introduction du tampon (4, 5) dans la cavité nasale et pour son retrait, caractérisé par le fait que, dans un plan auquel l'axe longitudinal du dispositif est perpendiculaire, le tampon (4, 5), avant son introduction dans la cavité nasale, a une section transversale généralement piriforme, ladite section ayant une largeur maximale (e—f) en dessous du point moyen de sa hauteur (c—d) et ladite hauteur (c—d) étant environ double de ladite largeur maximale (e—f) si bien que le tampon (4, 5) est adapté à l'absorption des liquides et à l'application d'une pression à la muquese traumatisée lorsqu'il est introduit dans la cavité.

2. Un dispositif comme revendiqué dans la revendication 1, caractérisé par le fait que la poignée (1, 2, 3) est ancrée dans le tampon absorbant les liquides (4, 5) et dépasse longitudinalement du tampon (4, 5) sur une distance suffisante pour pouvoir être saisie à l'extérieur de la cavité nasale lorsque le tampon (4, 5) y est logé, ce qui permet une manipulation facile du tampon (4, 5) et son introduction dans la cavité et son retrait.

3. Un dispositif comme revendiqué dans l'une ou l'autre des revendications précédentes, caractérisé par le fait que le tampon (4, 5) est conçu pour se dilater globalement de façon élastique lorsqu'il absorbe un liquide afin d'accroître la pression appliquée par le tampon à la muqueuse nasale lorsque le liquide est absorbé.

4. Un dispositif comme revendiqué dans l'une quelconque des revendications précédentes, caractérisé par le fait que le tampon (4, 5) comprend une partie centrale (4) en coton hydrophile entourée d'au moins une couche (5) de gaze de coton.

0 084 960

FIG. 1

FIG. 2

FIG. 3